# EUROPEAN PATENT APPLICATION

(11) **EP 3 626 227 A1**
(43) Date of publication of application: **25.03.2020**
(21) Application number: 17909830.6
(22) Date of filing: 17.05.2017
(51) Int. Cl.: A61K 8/67, A61K 8/58, A61K 8/9783, A61K 8/92, A61Q 19/08

(54) **COSMETIC COMPOSITION WITH AN OIL TEXTURE, USE AND METHOD FOR PREVENTING AND/OR TREATING SIGNS OF EXTRINSIC AGING AND CUTANEOUS STRESS**

(71) Applicant: Natura Cosméticos S.A., 05106-000 São Paulo - SP (BR)
(72) Inventor: CAROLLO MONCAYO, Priscila, CEP-05106-000 São Paulo - SP (BR); RODRIGUES DE PAULA, Leonardo, CEP-05106-000 São Paulo - SP (BR); ARANDAS MONTEIRO E SILVA, Silas, CEP-05106-000 Soã Paulo - SP (BR); CARVALHÃES LAGO, Juliana, CEP-05106-000 São Paulo - SP (BR); DE OLIVEIRA REIS, Eduardo Alexandre, CEP-05106-000 São Paulo - SP (BR); BAIONE DE MOURA, Soraya, CEP-13208-703 Jundiaí - SP (BR)
(74) Representative: Simmons & Simmons
(86) International application number: PCT/BR2017/050117
(87) International publication number: WO 2018/209411

(57) **Abstract**

The present invention relates to ultra-light oil texture cosmetic compositions suitable for being applied on the facial skin, as well as the use thereof in the treatment and prevention of cutaneous stress and signs of extrinsic aging, particularly caused by external pollution aggressors.

## Description

### Field of the Invention

The present invention relates to ultra-light oil texture cosmetic compositions suitable for being applied on the facial skin, as well as the use thereof in the treatment and prevention of cutaneous stress and signs of extrinsic aging, particularly caused by external pollution aggressors.

### Background of the invention

The environment and our lifestyle affect our skin's health and quality. Throughout the day the skin is exposed to various types of aggressors, particularly from pollution, which unbalances and accelerates aging and provokes cutaneous aging.

Natural or chronological aging is the physiological decay process that takes place in our body over the years, also designated as intrinsic aging. The skin aging process induced by environmental factors is the so-called premature or extrinsic aging and can be distinguished from chronological or intrinsic aging by the characteristic signs of each type of aging. In extrinsic aging the signs are more pronounced on the skin, such as thicker and rougher skin, formation of dark spots and stains, telangiectasia (presence of tiny dilated blood vessels on the skin) in addition to sagging and deeper wrinkles.

Exposure to environmental factors, including air pollution, is responsible for skin damage leading to extrinsic aging and cutaneous stress, as listed below:
1. Free radical generation causes the activation of receptors on the skin surface that would be deactivated under normal conditions, causing the activation of factors that lead to a reduced synthesis of collagen, which is one of the main skin supporting proteins, as well as the oxidation of skin components that can lead to DNA damage.
2. Activation of aryl hydrocarbon receptor (AhR), which is a damage-causing receptor, can be triggered by the interaction between toxic components deposited on the surface of pollution particles and the skin, promoting a cascade of damage, such as synthesis of metalloproteinases responsible for the degradation of extracellular matrix proteins, including collagen and elastin, as well as stimulation of genes that promote skin pigmentation disorders and microdamage processes.
3. Decreased skin barrier quality due to the oxidation of important components such as squalene and vitamin E by pollution, as well as damage caused by the effects of UV radiation and air-conditioned environments (low air humidity).
4. Increased microdamage processes caused by cytosines that intensify processes of forming lines and wrinkles on the skin.

Although the body itself has defense systems intended to neutralize or remove environmental damage, detection of such damage under normal physiological conditions even in healthy organisms does not reach 100% efficiency, resulting in an imbalance between defenses and the resulting damage, which requires the use of complementary treatment systems.

Thus, there remains a need for cosmetic compositions to aid in the prevention and treatment of environmental damage that will invariably lead to extrinsic aging and cutaneous stress.

### Description of the invention

Considering the main imbalances caused by pollution aggressors on the skin, which accelerate the process of extrinsic aging and cutaneous stress, an ultra-light oil texture cosmetic composition suitable for being applied on the facial skin has been developed to provide vitality and radiance to skin stressed by pollution and daily aggressions.

The ultralight oil texture cosmetic composition of the present invention acts on the eight major skin repair and protection mechanisms to slow down the appearance of extrinsic aging signs:
1. generation of free radicals that cause oxidative stress on the skin;
2. damage to skin DNA;
3. unbalance of the skin's natural antioxidant protection; and
4. increased microdamage caused by cytosines that intensify the formation of wrinkles;
5. Increase in the collagen degrading enzymes;
6. increased in elastin degrading enzymes;
7. damage to natural components of the skin barrier;
8. activation of damage-causing skin receptors.

In accordance with the present invention, the oil texture cosmetic form is suitable for application on the facial skin and was developed from volatile, short chain emollients that are rapidly absorbed into the skin with non-essential fatty acids and essences which, via the lipidation process of the skin improves its properties leaving it immediately soft and velvety.

The barrier function of the skin is mediated by intercellular bilayers in the stratum corneum. Cholesterol, ceramides, as well as non-essential and essential fatty acids play a key role in the formation of bilayers. Stratum corneum lipids are composed of 40% ceramides, 25% cholesterol and 20% free fatty acids. Incorporation of physiological lipids applied to the lipid barrier follows two paths:
a) direct incorporation into the membrane domain of the stratum corneum;
b) lipids traverse an intercellular route in the stratum corneum and are incorporated into the lower cell layers of the granular stratum.

The epidermis can internalize and physiologically process these lipids.

Thus, volatile emollients can provide a skin lipidizing effect, which is of importance to skin exhibiting discomfort of cracking and stiffness. The lipidizing effect also plays a significant and priority role in skin plasticity. In addition, lipid-rich formulations improve skin distensibility, while low lipid creams and gels have a more pronounced effect on skin hydration.

The ultralight oil texture of the cosmetic composition according to the present invention is achieved by selecting groups of ingredients that will ensure that the composition blends facially with the skin and by a lipidation process, improving the sensory properties thereof and causing it to be immediately soft and velvety.

Thus, in a first aspect, the present invention relates to an ultra-light oil textured cosmetic composition suitable for being applied on the facial skin, the benefits of which, as discussed herein, being guaranteed by a synergistic combination containing the following ingredients:
(a) at least one siliconized *Carpotroche brasiliensis* ester (sapucain), particularly hydroxycyclohexyl ethyltrisiloxane sapucainhate;
(b) at least one polyphenol, particularly from *Zingiber officinale* (ginger);
(c) at least one vitamin, particularly tocopheryl acetate;
(d) at least one synthetic molecule capable of stimulating CYP1A1, POMC, IL-6 and MMP-1 genes, particularly selected from benzylidene dimethoxydimethylindanone;
(e) at least one *Vitis vinifera* oil, particularly derived from the seed; and
(f) at least one essential oil, being particularly selected from *Lavandula angustifolia, Perlagonium graveolens, Rosmarinus officinalis, Dipteryx odorata* e/ou *Copaifera officinalis.*

The composition according to the present invention may further comprise cosmetically acceptable excipients known in the art.

In another aspect, the present invention further relates to the use of the ultralight oil texture cosmetic composition according to the present invention in the manufacture of a cosmetic product in the form of a facial oil for the treatment and prevention of signs of extrinsic aging and cutaneous stress, particularly caused by external pollution aggressors.

The composition according to the present invention acts to protect against damage caused by daily aggressions, relieving stress on the skin particularly that caused by dryness, protects the skin against pollution-induced damage, thereby recovering softness and radiance of the skin, giving it an even texture, stimulating the natural defenses, restoring vitality and balance of the skin.

In another aspect, the present invention further relates to a method of preventing and/or treating the signs of extrinsic aging and cutaneous stress consisting of applying on the skin an effective amount of the ultralight oil texture cosmetic composition according to the present invention.

The following examples, without limitation, illustrate the compositions according to the present invention as well as their effects, as described herein.

### EXAMPLES

### Example 1. Ultralight oil texture cosmetic composition according to the present invention

The ultralight oil texture cosmetic composition according to the present invention was prepared as depicted in the following table.

**Table 1. Ultralight oil texture cosmetic composition according to the present invention**

| Ingredient | (%) |
|---|---|
| BHT | 0.05 |
| Caprylic/capric triglyceride | 61.45 |
| Dicaprylyl carbonate | 19 |
| Dodecane | 11 |
| *Elaeis guineensis* oil | 4 |
| benzylidene dimethoxydimethylindanone | 0.5 |
| hydroxycyclohexyl ethyltrisiloxane sapucainate | 0.3 |
| Ingredient | (%) |
| tocopheryl acetate | 1 |
| *Vitis vinifera* oil | 2 |
| *Zingiber officinale* extract | 0.2 |
| *Dipteryx odorata* essential oil | 0.024 |
| *Lavandula angustifolia* essential oil | 0.119 |
| *Perlagonium graveolens* essential oil | 0.119 |
| *Rosmarinus officinalis* essential oil | 0.214 |
| *Copaifera officinalis* essential oil | 0.024 |

### Example 2. In vitro efficacy of anti-stress and anti-pollution effects

The combined action of ingredients selected for the ultra-light oil texture cosmetic composition according to the present invention in the protection against the main biological damages caused by pollution and other external aggressors on the skin was studied. The following damage markers were studied:

| Marker | Function | What effect was perceived on the skin? |
|---|---|---|
| Metalloproteinase 1 | Enzyme involved in extracellular matrix breakdown, particularly, collagen. | Wrinkles and sagging. It causes skin structural proteins to breakdown. |
| Metalloproteinase 3 | Enzyme involved in extracellular matrix breakdown: Collagen and elastin. | Wrinkles and sagging. It causes skin structural proteins to breakdown. |
| Proinflammatory cytokine | Protein mainly synthesized in areas of acute and chronic inflammation and secreted into the extracellular environment (outside the cell). | It induces an inflammatory response, which can cause damage to dermal proteins. |
| Marker | Function | What effect was perceived on the skin? |
| Antioxidant activity, stimulation of endogenous antioxidants and DNA protection. | It neutralizes the action of free radicals resulting from natural physiological processes and processes induced by external aggressors. | It can cause protein degradation, breakdown of lipid cell walls and DNA damage. |

The ultralight oil texture cosmetic composition according to the present invention was tested using *in vitro* techniques in order to understand its mechanisms of action and the following results were obtained:
- it reduces the synthesis of IL6 by 74% as compared to cells exposed to damage (microdamage reduced by 74%);
- it reduces the synthesis of MMP1 by 33% as compared to cells exposed to damage (collagen- and elastin-degrading enzymes reduced by 33%)
- it reduces the synthesis of MMP3 by 33% as compared to cells exposed to damage (it reduces enzymes that degrade dermal proteins
- it has 91% antioxidant protection (protects free radical action by 91%);
- it stimulates the skin's natural antioxidants;
- it protects the DNA from damage caused by external factors;

### Example 3. Barrier strengthening

The skin barrier strengthening was assessed using an objective, non-invasive methodology designated TEWL ("Transepidermal Water Loss"), which indicates the skin barrier integrity through measurements of transdermal water loss that represents one of the primary barrier functions, that is, in addition to preventing the entry of microorganisms, chemicals and various forms of radiation, it prevents the body from losing water and nutrients while maintaining the skin impervious, which is essential for the proper functioning of the organism. In this evaluation measurements were taken prior to application of the product in order to provide information on the baseline condition of the skin, and after 14 and 28 days of home use of the product.

Applications are made on the forearm, which is a region less affected by external factors and being more protected from sun exposure. A significantly positive effect of barrier strengthening was seen at all times evaluated.

The table below shows the percentage of barrier strengthening provided by the ultra-light oil texture cosmetic composition according to the present invention, when comparing the baseline condition of the skin (prior to application thereof), as well as the percentage of volunteers in which this effect was observed.

**Table 2. Skin barrier strengthening results**

| Days of Use | % improvement | % of volunteers who showed improvement |
|---|---|---|
| 14 | 5.4 | 89.3 |
| 28 | 7.5 | 92.9 |

The product achieved significant improvement in skin barrier strengthening after 14 and 28 days of treatment.

### Example 3. Anti stress action

The anti-stress action was evaluated using a non-invasive objective methodology designated "Cutaneous Stress Reduction Test", which indicates the stress response of a given substrate when exposed to different adverse conditions (different temperature and moisture conditions). The test considered adhesion of the stratum corneum on a surface having specific mechanical properties (coverslip substrate). Subsequently, the product was applied on skin adhered to the substrate and exposed in a chamber under controlled moisture and temperature conditions (temperature of 22°C; low Relative Humidity - 20 RH; and high Relative Humidity 50 RH). The obtained results were considered in relation to the control condition (stratum corneum without any product applied thereon). Significant positive effect of cutaneous stress reduction was obtained under the different moisture conditions tested.

The table below shows the cutaneous stress reduction performance of the ultralight oil texture cosmetic composition according to the present invention.

**Table 3. Cutaneous stress reduction performance**

| Cutaneous stress reduction (Relative Humidity 20%) - versus Control | Cutaneous stress reduction (Relative Humidity 50%) - versus Control |
|---|---|
| 20.5 % | 56.4 % |

The ultralight oil texture cosmetic composition according to the present invention significantly reduced the cutaneous stress caused by dryness under different relative humidity conditions.

### Example 4. Consumer sensory research

In order to understand the acceptability and pleasantness profile as well as the specific perception of some product attributes, it is sometimes necessary to subject them to sensory testing in use with consumers.

In this study participants used the ultralight oil texture cosmetic composition according to the present invention for 14 days and answered a sensory attribute evaluation questionnaire at different times: T1 (after application of the product on day 1 at the institute), T7 (after 7 days of use) and T14 (after 14 days of use of the product). The panel of volunteers included 120 women (potential final consumers of the product) residing in the city of São Paulo (economic classes A and B; aged from 25 to 60 years) exposed to cutaneous stress conditions (smokers, exposed to pollution, having habits involving sleeping for a few hours only - self-reporting to live under stressful conditions). In this study it was imperative to create a panel with comparable representativeness of the different skin types: dry, normal, oily/mixed skin.

The ultralight oil texture cosmetic composition according to the present invention has met the sensory metrics of the action pattern (pleasantness - Top 2 Box and acceptability - Top 3 Box). The main attribute perception results with respect to the ultralight oil texture cosmetic composition according to the present invention were:
- immediate relief of skin stress;
   - 91% of women felt immediate stress relief;
   - soft and moisturized skin;
- revitalized skin feel;
- radiant skin with vitality, luminous and more vigorous skin;
- skin protected for any event;
- it forms a shield on the skin.
   - revitalized/rebalanced skin/ it restores skin vitality and balance;
- it improves the overall appearance of the skin;
- it is ideal for the skin type;
- it did not cause acne/pimple;
- it slows down/decelerates the signs of aging;
- it recovers skin radiance immediately;
- ultra light oil texture that blends easily with the skin;
- it leaves the skin velvety;
- it leaves the skin with a dry touch;
- it provides an even texture to the skin; and
- feeling of relaxation.

### Example 5. Product safety

To assess the absence of sensations and signs of discomfort, safety evaluation tests were performed under the supervision of dermatologists. Safety of the ultralight oil texture cosmetic composition according to the present invention was ensured by the following tests.

### Acceptability in use

This study consists of volunteers using the test product at home under real use conditions (the same condition as shown on the product packaging) in order to verify whether the product is well accepted by the volunteers' skin (panel contemplating volunteers having normal, dry and mixed/oily skin). Dermatological and/or ophthalmic clinical evaluations are performed prior to the use of the test product (to verify the volunteer's baseline skin and eye condition and whether he/she is able to participate in the study) and after 21 days of use to test product to ensure that the product did not promote any changes on the skin and eye health.

As a result, the ultralight oil texture cosmetic composition according to the present invention was shown to be safe for use in humans, causing no irritation or sensitization reactions in the analyzed sample of volunteers, the products being declared as dermatologically tested.

### PC5

This study aims to evaluate the product safety under slightly maximized conditions, taking into account the amount of product applied and the occlusion procedure, and with supervised use for each application in order to capture possible feelings of discomfort and/or clinical signs that the product may have a cumulative effect on the skin.

As a result, for the ultralight oil texture cosmetic composition according to the present invention, no volunteer exhibited skin reactions and they can be considered to have very good skin compatibility.

### HRIPT

This study aims to evaluate the product safety under maximized conditions, taking into account the amount of product applied and the occlusion procedure (use of occlusive and semi-occlusive dressings) so as to capture possible feelings of discomfort and/or clinical signs that the product may have a cumulative effect on the skin, characterizing possible irritation and/or sensitization reactions.

As a result, for the revitalizing concentrate product, no volunteers exhibited skin reactions and the product can be deemed safe under the assessed conditions as it did not induce primary or cumulative skin irritation or skin sensitization in the study group.

### Photo test

This study aims to evaluate the product safety under conditions of sun exposure, taking into account the amount of product applied and the occlusion procedure (use of occlusive and semi-occlusive dressings) so as to capture possible feelings of discomfort and/or clinical signs that the product may have a cumulative effect on the skin and by solar exposure, characterizing possible irritation and/or sensitization reactions.

As a result, for the ultralight oil texture cosmetic composition according to the present invention, no volunteers exhibited skin reactions and the product can be deemed safe under the assessed conditions as it did not induce primary or cumulative skin photo irritation or skin photosensitization in the study group. Comedogenicity

This study consists of volunteers using the test product at home under real use conditions (the same condition as shown on the product packaging) in order to verify whether the product is comedogenic, that is, whether it has features that favor the appearance of acne/comedones (panel contemplating volunteers having acne-prone oily skin). Clinical dermatological evaluations are performed prior to the study on day 1 and on day 28 of the study (to assess the baseline skin condition by counting comedones and acne lesions to assess the participant's acne/comedogenic profile). Then, the participant starts using the product and after 28 days of use, she/he returns to the doctor for a new assessment of the voluntary participant's skin to be made (with a new count of comedones and acne lesions). Absence of a significant increase of acne lesions/comedones means that the product does not have comedogenic potential.

As a result, the ultralight oil texture cosmetic composition of the present invention was shown to be comedogenic.

From the teachings provided in the text and the examples presented herein the person skilled in the art will readily appreciate the advantages of the invention and will be able to propose variations and equivalent embodiment alternatives without departing from the scope of the invention as defined in the appended claims.

## Claims

1. ULTRALIGHT OIL TEXTURE COSMETIC COMPOSITION **characterized in that** it comprises:
(a) at least one siliconized *Carpotroche brasiliensis* ester;
(b) at least one polyphenol;
(c) at least one vitamin;
(d) at least one synthetic molecule capable of stimulating CYP1A1, POMC, IL-6 and MMP-1 genes;
(e) at least one *Vitis vinifera* oil, and
(f) at least one essential oil having antioxidant properties.
(g) optionally, cosmetically acceptable excipients.

2. The COMPOSITION of Claim 1, **characterized in that** it comprises:
(a) hydroxycyclohexyl ethyltrisiloxane sapucainate;
(b) *Zingiber officinale* polyphenol;
(c) tocopheryl acetate;
(d) benzylidene dimethoxydimethylindanone;
(e) *Vitis vinifera* seed oil;
(f) at least one essential oil selected from *Lavandula angustifolia, Perlagonium graveolens, Rosmarinus officinalis, Dipteryx odorata* e/ou *Copaifera officinalis.*
(g) optionally, cosmetically acceptable excipients.

3. The USE of the cosmetic composition of claim 1 or 2, **characterized in that** it is in the manufacture of a cosmetic product useful in the treatment and prevention of signs of extrinsic aging and cutaneous stress.

4. The USE of Claim 3, **characterized in that** it is a facial oil.

5. A METHOD OF PREVENTING AND/OR TREATING SIGNS OF EXTRINSIC AGING AND CUTANEOUS STRESS **characterized in that** it consists of applying on the skin an effective amount of the cosmetic composition of claim 1 or 2.
